# EUROPEAN PATENT APPLICATION

(11) **EP 4 325 206 A1**
(43) Date of publication of application: **21.02.2024**
(21) Application number: 22190899.9
(22) Date of filing: 18.08.2022
(51) Int. Cl.: G01N 21/64, C12Q 1/686

(54) **METHOD FOR COMPENSATING DEFECTIVE PARTITIONS OF A MICROFLUIDIC CHIP**

(71) Applicant: F. Hoffmann-La Roche AG, 4070 Basel (CH); Roche Diagnostics GmbH, 68305 Mannheim (DE)
(72) Inventor: Pattje, Wouter Johannes, 6343 Rotkreuz ZG (CH)
(74) Representative: Altmann Stößel Dick Patentanwälte PartG mbB

(57) **Abstract**

A method for compensating defective partitions of a microfluidic chip (112) and a sample processing system (110) for performing the method are disclosed. The microfluidic chip (112) comprises at least one array of partitions (114). The method comprises the following steps:
a) generating image data of the microfluidic chip (112) comprising a sample by imaging the microfluidic chip (112) by using at least one imaging device (116);
b) providing at least one mask (136, 137) for at least one area of partitions to at least one data processing unit (118);
c) applying the mask (136, 137), by using the data processing unit (118), to one or more of the image data, at least one feature extracted from the image data, or at least one value extracted from the image data, thereby generating masked data, wherein the applying of the mask comprises masking each partition of the array (114) depending on the mask;
d) generating at least one analytical result for the sample using said masked data.

## Description

### Technical Field

The present invention relates to a method for compensating defective partitions of a microfluidic chip and a sample processing system for performing the method. Further, the invention relates to a computer program and a computer-readable storage medium for performing the method. The method and devices may specifically be used in the field of medical or chemical laboratories, in particular in the field of automated in vitro diagnostic (IVD) sample processing. Other fields of application of the present invention, however, are feasible.

### Background art

There are a number of analyses, which uses a multitude of single analyses to produce an analytical result. For that purpose, a solution containing analytes of interest is physically distributed into a multitude of individual reaction partitions. Examples are digital polymerase chain reaction (dPCR), d-isothermal nucleic acid amplification, but also other analysis may be conducted in or on arrays such as sequencing , digital cell culturing, etc.. Such a multitude of single analyses are frequently conducted in so called arrays (of partitions). In order to produce valid results and/or results with a high precision or high accuracy or high confidence it is important, that all, or at least a significant ratio of all single analyses in the array, also called "elements" or "partitions" of the array, behave the same way. It is therefore important to make sure only valid elements of the arrays are used or considered in the analysis or used to calculate a result.

Such arrays are frequently produced with technologies used for micro structuring, such as e.g. injection molding, photo-structuring, micro-embossing, printing, self-assembly etc. all these technologies have a risk to produce not a 100% yield. In other words there may be always a fraction of elements in the array which will not produce a correct result.

Injection molded microfluidic devices may suffer from quality issues in the manufacturing process at specific spatial locations due to the nature of the manufacturing process. These kind of quality issues or defects become obvious during the development of the manufacturing process. Even though optimization of the process should eliminate such defects, it may be reasonable to accept a certain amount of defects to increase production yield and decrease production cost. If these structural defects negatively affect the analytical results of the analysis conducted on those microfluidic devices, methods have to be defined to compensate these effects. In the case of a diagnostic digital assay conducted in a microfluidic array of partitions, it is crucial to maintain highest result quality. For this reason, a method for properly excluding areas with defects from the analysis is of high importance. Also, it is of high importance not to exclude large areas with no defects, since the quality of the result depends on the number of individual reactions, and should be as large as possible.

Common type of such defect recognition system are based on pattern recognition and/or artificial intelligence applied on the entirety of the microfluidic area. However, these systems tend to produce a certain amount of false positive and false negative results. Since the affected areas by injection molding processes are at least partly very well-known in terms of size and location, any pattern recognition method applied on the entirety of the injection molding part will likely produce false negatives in the affected area and false positives in the not affected area.

In addition, applying pattern recognition or artificial intelligence methods to recognize affected areas may result in erratic patterns from e.g. lot-to-lot of molded parts. If the information of lot specific areas to exclude due to defects has to be encoded on the injection molded part itself, e.g. on a bar code, the size to store that information may be limited to a few Bytes, which makes encoding of complex data (e.g. a large amount of x,y coordinates or indexes) impossible.

For example, EP 2 761 585 B1 describes a method for calibrating a biological instrument. The method comprises the steps of acquiring an image of at least one biological sample array, determining a first region of interest within the image, wherein the first region of interest comprises a first plurality of locations on the at least one biological array; and identifying within the first region of interest, a plurality of image elements associated with each of the first plurality of locations on the at least one biological array.

US 9 434 987 B2 describes a cartridge for an optical analysis, including a supporting body, having a first face and a second face opposite to one another; a plurality of wells adapted to receive a biological solution to be analyzed, the wells extending in the supporting body on the first face; at least one biocompatible layer extending inside each well; an anti-reflection layer extending on the first face outside the wells; and a reflection layer extending inside each well.

US 2019/0113457 A1 describes methods, devices, assemblies, and systems for dispensing into the wells of a multi-well device and imaging such wells from multiple Z-planes. Also provided are methods, devices, assemblies, and systems for processing cell-containing wells of a multi-well device identified through the use of multi-Z imaging.

US 8 927 465 B2 describes a process for making a micro-array. The process comprises the step of depositing a population of microbeads on a substrate having at least one fiducial. The population being comprised of at least two sub-populations, preferably multiple sub-populations, each comprising a known active agent capable of specific binding with at least one target analyte. The said subpopulations are deposited sequentially and at discrete periods of each other. The process also comprises the step of making images of the substrate after deposition of each subpopulation. The images are then compared using the fiducial as a reference to thereby determine the location of each microbead and to identify the subpopulation, and its known active agent, based on differences between each image. Also disclosed in a system for using the microarray.

US 2019/0244345 A1 describes an automated analysis instrument which operates to detect inadequate dispensation of a fluidic substance on a tray. The instrument includes an image capturing device to capture an image of at least a portion of the tray including a receptacle portion and a surrounding portion around the receptacle portion. The instrument then identifies the surrounding portion of the at least the portion of the tray in the image, evaluates color components of the image corresponding to the surrounding portion of the at least the portion of the tray, and determines whether the fluidic substance is present on the surrounding portion of the at least the portion of the tray based on at least one of the color components.

In other technical fields further techniques for defect recognition are known such as described in US 10 891 520 B2, US 10 964 004 B2, WO 2020/255498 A1, US 2017/0323435 A1, US 10 891 725 B2, US 2020/0020094 A1, US 10 600 175 B2.

### Problem to be solved

It is therefore desirable to provide methods and devices which at least partially address above-mentioned technical challenges. Specifically, a method for compensating defective partitions of a microfluidic chip and an automated processing system shall be proposed which allow to consider non-valid elements in an array of single analyses with high reliability.

Specifically, a method and means shall be proposed, to use microfluidic chips to produce analytical results, where the microfluidic chips have a significant fraction of defective partitions, e.g. due to suboptimal manufacturing processes, without compromising the analytical result significantly. Whereas otherwise, without using this method and means, these microfluidic chips with a significant fraction of defective partitions could not be used, would need to be discarded, or which, if be used, would compromise the results generated with these microfluidic chips.

### Summary

This problem is addressed by a method for compensating defective partitions of a microfluidic chip and a sample processing system with the features of the independent claims. Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect, a method for compensating defective partitions of a microfluidic chip is proposed. The microfluidic chip comprises at least one array of partitions.

The term "microfluidic chip" as used herein is a broad term and is to be given its ordinary and customary meaning to a person of ordinary skill in the art and is not to be limited to a special or customized meaning. The term specifically may refer, without limitation, to at least one substrate comprising at least one microfluidic device. The microfluidic chip may be configured for handling volumes ranging from picoliter-to-millilitre range within the microfluidic structures of the microfluidic chip. The microfluidic chip may comprise a plurality of microfluidic devices. The microfluidic device may comprise at least one inlet opening and at least one outlet opening. The microfluidic device may be or may comprise a flow channel connecting said inlet opening and said outlet opening and defining a flow direction from the inlet opening through the flow channel to the outlet opening. The microfluidic device may comprise at least one top layer and at least one bottom layer. For example, the microfluidic device may comprise at least one substrate and at least one cover, e.g. in the form of a plate or foil. The top layer and the bottom layer may be attachable to each other. The flow channel may be arranged between the top layer and the bottom layer. For example, the microfluidic chip may be designed as described in EP 3 610 947 B1, or EP 3 714 977 A1.

The term "partition", also denoted as microfluidic partition or element, as used herein is a broad term and is to be given its ordinary and customary meaning to a person of ordinary skill in the art and is not to be limited to a special or customized meaning. The term specifically may refer, without limitation, to a microwell or a nanowell. The microfluidic chip may be a well plate, e.g. a microwell plate or a nanowell plate, comprising featuring several hundreds or even thousands of wells. The partitions may be provided by the top layer or the bottom layer. The microfluidic device may comprise an array of partitions. The microfluidic device may be also denoted as a microarray. The array of partitions may be in fluidic communication with the flow channel, an inlet and an outlet to introduce or evacuate fluids. The array may be a grid and/or matrix of partitions, such as a rectangular grid. For example, the rectangular grid may be interleaved, e.g. the grid may be a hexagonal grid. Other arrays are possible. The microfluidic chip comprises at least one array of partitions. For example, the microfluidic chip may comprise a plurality of arrays of partitions. For example, the amount of partitions of each array may be the same. A location of each of the partitions may be described by a tuple of x and y coordinate indices and/or an absolute index. The array may comprise rows and columns. The array may be rectangular having m rows and n columns, with m, n, independently, being positive integers. The volume of a partition might be most preferred in a range of 0.1 to 5nl, less preferred 0.01 to 50nl, least preferred 0.001 to 1ul.

The microfluidic chip may be fabricated using technologies used for micro structuring, such as e.g. injection molding, photo-structuring, micro-embossing, printing, self-assembly etc. The method may comprise providing of the microfluidic chip. The method may comprise at least one manufacturing process. The microfluidic chip may be fabricated using at least one technology used for micro structuring. The microfluidic chip may be fabricated using at least one technology selected from the group consisting of injection molding, photo-structuring, micro-embossing, printing, self-assembly and the like. For example, the manufacturing process comprises injection molding.

Each of the partitions may constitute a reaction area. The microfluidic chip may be configured for a multitude of single analyses using the arrays of partitions. The term "single analyses" as used herein is a broad term and is to be given its ordinary and customary meaning to a person of ordinary skill in the art and is not to be limited to a special or customized meaning. The term specifically may refer, without limitation, to at least one generally arbitrary biochemical, chemical, physical or biological reaction. The multitude of single analyses may comprise a multitude of parallel reactions. Examples of biochemical reactions may be digital polymerase chain reaction (dPCR), d-isothermal nucalic acid amplification, and d-enzymatic reaction, digital enzyme-linked immunosorbent assay (ELISA), and other analysis such as sequencing, digital cell culturing, and the like. For example, the microfluidic chip may be a dPCR chip.

The method may comprise at least one partitioning step in which a sample is filled into the microfluidic chip. For example, a solution comprising analytes of interest may be physically distributed into the partitions and physically separated, that no (significant) diffusions can take place between each of the partitions. The method further may comprise providing at least one reagent to analyze said sample on said microfluidic chip. For example, the microfluidic chip is configured for digital PCR or biochemical assaying of a sample provided, e.g. in the form of a reaction mixture, to each of the partitions by means of the microfluidic devices. The term "sample" as used herein is a broad term and is to be given its ordinary and customary meaning to a person of ordinary skill in the art and is not to be limited to a special or customized meaning. The term specifically may refer, without limitation, to a material suspected of containing an analyte of interest. The sample can be derived from any biological source, such as a physiological fluid, including, blood, saliva, ocular lens fluid, cerebral spinal fluid, sweat, urine, milk, ascites fluid, mucous, synovial fluid, peritoneal fluid, amniotic fluid, tissue, cells or the like. The sample can be pretreated prior to use, such as preparing plasma from blood, diluting viscous fluids, lysis or the like; methods of treatment can involve filtration, distillation, concentration, inactivation of interfering components, and the addition of reagents. A sample may be used directly as obtained from the source or following a pretreatment to modify the character of the sample, e.g. after being diluted with another solution or after having being mixed with reagents e.g. to carry out one or more diagnostic assays like e.g. clinical chemistry assays, immunoassays, coagulation assays, nucleic acid testing, etc.. The term "sample" as used herein is therefore not only used for the original sample but also relates to a sample which has already been processed such as pipetted, diluted, mixed with reagents, enriched, having been purified, having been amplified and the like. As used herein, the term "analyte" may refer to a compound, a molecule (e.g. a nucleic acid) or a microorganism to be detected or measured. The term "reagent" as used herein is a broad term and is to be given its ordinary and customary meaning to a person of ordinary skill in the art and is not to be limited to a special or customized meaning. The term specifically may refer, without limitation, to a composition required for treatment of a sample.

Reagents may be any liquid, e.g. a solvent or chemical solution, which needs to be mixed with a sample and/or other reagent in order e.g. for a reaction to occur, or to enable detection, especially if an analyte (molecule) is present in a partition. A reagent may be for example a diluting liquid, including water, it may comprise an organic solvent, it may comprise a detergent, it may be a buffer. Reagents may also be dry reagents adapted e.g. to be dissolved by a sample, another reagent or a diluting liquid. A reagent in the more strict sense of the term may be a liquid solution containing a reactant, typically a compound or agent capable e.g. of binding to or chemically transforming one or more analytes present in a sample. Examples of reactants are enzymes, enzyme substrates, conjugated dyes, protein-binding molecules, nucleic acid binding molecules, primers and probes, antibodies, chelating agents, promoters, inhibitors, epitopes, antigens, etc.. For example, for conducting a dPCR assay, a sample comprising an analye and dPCR reagents (including dNTPs, primer, probes, Polymerase, buffer-substances and detergents) is introduced to the microfluidic chip, e.g. by pipetting the aqueous dPCR reaction mixture into the inlet opening of the microarray.

The method may be performed by a sample processing system, e.g. a processing system as described in a further aspect below. The sample processing system may be configured for performing at least one processing step on the sample. The sample processing system may be configured for performing a workflow, consisting out of a multitude of processing steps, for processing the sample. The term "processing a sample" as used herein is a broad term and is to be given its ordinary and customary meaning to a person of ordinary skill in the art and is not to be limited to a special or customized meaning. The term specifically may refer, without limitation, to any treatment of the sample and for handling any objects and data required to analyze the sample. It may comprise transferring, aliquoting, isolating, purifying, incubating, reacting a sample or combining a reagent with a sample, analyzing and the like. The term "processing step" as used herein is a broad term and is to be given its ordinary and customary meaning to a person of ordinary skill in the art and is not to be limited to a special or customized meaning. The term specifically may refer, without limitation, to at least one physically executed step, e.g. of a workflow, for processing the sample. The workflow comprises at least one, usually a plurality of processing steps. For example, the workflow may comprise a chain of steps to conduct a dPCR method. The sample processing system may comprise at least one analytical processing system. The sample processing may further comprises a pre-analytical processing system and/or a post-analytical processing system. The sample processing system may comprise at least one instrument such as one or more pre-analytical, analytical, and post-analytical instrument. A pre-analytical instrument can usually be used for the preliminary processing of samples. An analytical instrument can be designed, for example, to use a sample or part of the sample and a reagent in order to produce a measurable signal, on the basis of which it is possible to determine whether the analyte is present, and if desired in what concentration. A post-analytical instrument can usually be used for the post-processing of samples like the archiving of samples. The sample processing system may comprise one or more of a pipetting device for pipetting a sample into the microfluidic chip, an aliquoting device for aliquoting samples, a centrifuging device for centrifuging samples, a heating device for heating a sample, a cooling device for cooling a sample, a mixing device for mixing a sample, a separation device for isolating an analyte of the sample, a storing device for storing samples, an archiving device for archiving samples.

The sample processing system may comprise at least one instrument for analyzing a sample. The analyzing may comprise performing at least analytical test on the sample. The method may comprise providing an instrument to analyze said sample on said microfluidic chip. For example, the instrument may be or may comprise at least one imaging device e.g. for fluorescence imaging the microarray. The term "to analyze" as used herein is a broad term and is to be given its ordinary and customary meaning to a person of ordinary skill in the art and is not to be limited to a special or customized meaning. The term specifically may refer, without limitation, to performing at least one analytical test such as determining at least one analyte in the sample and/or at least one parameter of the sample and/or the analyte. The analysis may comprise generating at least one analytical result such as a quantitative and/or qualitative result for the sample. This analytical results may be one or more of, a result for concentration of a distinct target analyte, a result for the presence or absence of an analyte, a result for a distinct copy-number-variation, a result for a distinct genetic mutation, a result for gene-expression, a result related to gene editing, etc.

For example, the processing system maybe configured to pipette a sample, mix a sample with reagents, transfer a reaction mix to a microfluidic device, distribute the sample to the partitions of a microfluidic chip, perform one or a set of incubation steps, e.g. execute a so called "PCR-profile" (being a set of multiple incubation steps), afterwards fluorescence image the microarray and calculating from the image-data an analytical result of the sample.

The method may be computer-implemented. The term "computer implemented" as used herein is a broad term and is to be given its ordinary and customary meaning to a person of ordinary skill in the art and is not to be limited to a special or customized meaning. The term specifically may refer, without limitation, to a method involving at least one computer and/or at least one computer network. The computer and/or computer network may comprise at least one processor which is configured for performing at least one of the method steps of the method according to the present invention. Preferably each of the method steps is performed by the computer and/or computer network. The method may be performed completely automatically, specifically without user interaction. The terms "automatically" and "automated" as used herein are broad terms and are to be given their ordinary and customary meaning to a person of ordinary skill in the art and is not to be limited to a special or customized meaning. The terms specifically may refer, without limitation, to a process which is performed completely by means of at least one computer and/or computer network and/or machine, in particular without manual action and/or interaction with a user. The sample processing system may be an automated processing system. The sample processing system may be configured for automatically processing samples.

As outlined above, it is known that microfluidic chips may suffer from quality issues in the manufacturing process at specific spatial locations due to the nature of the manufacturing process. The manufacturing process may result in defect and/or non-valid partitions of the microfluidic chip. These defect and/or non-valid partitions would negatively affect the analytical results if the analysis is performed on those defect partitions. However, in order to increase production yield and decrease production cost it may be beneficial to accept a certain amount of defects. The method according to the present invention may allow for reliably excluding areas with defects from the analysis. The method according to the present invention may further allows for not excluding large areas with valid partitions (partitions with no defects). Excluding large areas with no defects may be disadvantageous since the quality of the analytical result depends on the analysis of an area, i.e. a number of individual reactions, is as large as possible. The location of the defects may be pre-known. For example, a usual location of the defects of microfluidic chips manufactured by a specific manufacturing process may be pre-determined such as from experimental studies or observations. For example, areas furthest away from an injection point, or border areas between different types of molding patterns may be affected with high probability. Also stamper defects, or stamper wearing may have effects on the quality of subsequently produced parts. Therefore, the method according to the present invention proposes to exclude such areas by default. The term "compensating defective partitions" as used herein is a broad term and is to be given its ordinary and customary meaning to a person of ordinary skill in the art and is not to be limited to a special or customized meaning. The term specifically may refer, without limitation, to taking into account defective partitions for analysis of the sample, e.g. by excluding defective partitions from analysis and/or correcting data obtained from the defective partitions.

The method steps may be performed in the given order or may be performed in a different order. Further, one or more additional method steps may be present which are not listed. Further, one, more than one or even all of the method steps may be performed repeatedly.

The method comprises the following steps:
a) generating image data of the microfluidic chip comprising a sample by imaging the microfluidic chip by using at least one imaging device;
b) providing at least one mask for at least one area of partitions to at least one data processing unit;
c) applying the mask, by using the data processing unit, to one or more of the image data, at least one feature extracted from the image data, or at least one value extracted from the image data, thereby generating masked data, wherein the applying of the mask comprises masking each partition of the array depending on the mask;
d) generating at least one analytical result for the sample using said masked data.

The term "image data" as used herein is a broad term and is to be given its ordinary and customary meaning to a person of ordinary skill in the art and is not to be limited to a special or customized meaning. The term specifically may refer, without limitation, to data recorded by using at least one imaging device, such as a plurality of electronic readings from the imaging device, such as the pixels of a camera chip. The term "imaging" as used herein is a broad term and is to be given its ordinary and customary meaning to a person of ordinary skill in the art and is not to be limited to a special or customized meaning. The term specifically may refer, without limitation, to providing a two-dimensional or three-dimensional representation of the arrays of partitions. The imaging may comprise of one or more of recording or capturing optical information, e.g. spatially resolved two-dimensional or even three-dimensional optical information. The imaging may comprise generating at least one image of the arrays of partitions. The term "imaging data" may refer also to data being processed during or after data acquisition. The data processing may include optical corrections, flat-field correction, image stitching, pixel binning and averaging and consolidation of pixel values, especially of pixels values related to an individual partitions (e.g. resulting in a table of brightness values for each partition).

The term "imaging device" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, a two-dimensional or even three-dimensional optical information. The imaging device may be at least one device selected from the group consisting of: at least one camera, at least one CCD camera, at least one CMOS camera, at least one RGB camera, at least one digital camera, at least one camera of a scanning microscope, at least one camera of a fluorescence microscope. The imaging device generally may comprise a one-dimensional or two-dimensional array of image sensors, such as pixels. The imaging device, besides at least one camera chip or imaging chip, may comprise further elements, such as one or more optical elements, e.g. one or more lenses, prisms, mirrors and / or pinholes. As an example, the imaging device may be a fix-focus camera, having at least one lens which is fixedly adjusted with respect to the camera. Alternatively, however, the imaging device may also comprise one or more variable lenses which may be adjusted, automatically or manually. The imaging device may also compromise an illumination, to illuminate the imaged area. In both optical paths, the illumination path and the detection path there might be optical filters e.g. designed to conduct a fluorescence measurement. Also means to switch between several filters may be provided e.g. in order to detect fluorescence at various excitation- and emission-wavelengths. The imaging device may comprise 1 or several stages, e.g. an x/y-stage, to acquire a multitude of images from the a least 1 array. The imaging device may comprise means for autofocusing. The imaging device may compromise optical shielding means to suppress the impact of ambient light to the measurement.

The method may comprise processing the image data. The processing may comprise extracting features and/or values from the image data.

The method may comprise preprocessing the image data. The pre-processing may comprise one or more of the following: at least one image correction; a filtering; a selection of at least one region of interest; a background correction; a decomposition into color channels; a decomposition into hue, saturation, and brightness channels; a frequency decomposition; a thresholding; creating a binary image, a flat-field-correction, a fluorescence crosstalk compensation, correction of optical distortions, stitching of multiple images, averaging images, excluding pixel-errors and deriving from at least one image file, where the brightness-values of pixels are listed, a partition data table, where the fluorescence values for all partitions are listed. The preprocessing of image data is preferably executed automatically based on a program, defining the preprocessing procedure.

The term "data processing unit" also denoted as "processor" or "processing device" as used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the data processing unit may be configured for processing basic instructions that drive the computer or system. As an example, the data processing unit may comprise at least one arithmetic logic unit (ALU), at least one floating-point unit (FPU), such as a math co-processor or a numeric co-processor, a plurality of registers, specifically registers configured for supplying operands to the ALU and storing results of operations, and a memory, such as an L1 and L2 cache memory. In particular, the data processing unit may be a multi-core processor. Specifically, the data processing unit may be or may comprise a central processing unit (CPU). Additionally or alternatively, the data processing unit may be or may comprise a microprocessor, thus specifically the processor's elements may be contained in one single integrated circuitry (IC) chip. Additionally or alternatively, the data processing unit may be or may comprise one or more application-specific integrated circuits (ASICs) and/or one or more field-programmable gate arrays (FPGAs) and/or one or more tensor processing unit (TPU) and/or one or more chip, such as a dedicated machine learning optimized chip, or the like. The data processing unit specifically may be configured, such as by software programming, for performing one or more evaluation operations. The data processing unit may be a server- or a cloud-based solution.

The term "mask" as used herein is a broad term and is to be given its ordinary and customary meaning to a person of ordinary skill in the art and is not to be limited to a special or customized meaning. The term specifically may refer, without limitation, to a data set for one or more of inclusion or exclusion or weighting partitions of an array, also denoted as mask data. The mask may comprise a weighting factor for each partition of the array. The mask may fully exclude non-valid partitions of the array, while keeping all valid partitions of the array. The weighting may comprise applying weighting factors. The weighting factor may be a value between 0 and 1. The mask may be an array specific mask or a mask per lot. The mask may comprise further information related to a single partition or a set of partitions. The mask may comprise further information, e.g. information about a reason for exclusion of the partition and/or information that partitions are excluded for one or more wavelength only.

The mask may comprise a list of partitions and/or a range of partitions. The range of partitions may comprise one or more of a list of rows of the array, a list of columns of the array, a rim-size, a list of array areas, a list of predefined geometrical figures, such as rectangles or triangles, or a combination thereof. For example, the mask may be a "simplified mask". The range of invalid partitions may be defined, e.g. by listing the whole rows or columns of the array to fully exclude or by providing a rim-size, e.g. a number of features, which have to be excluded from all microfluidic chips or by providing a predefined of mask. This may allow e.g. a reduction of data to store a mask, reduction of effort to process a mask, reduction of effort to handle masks.

The providing of the mask may comprise retrieving and/or generating the mask. The mask may be pre-determined or may be generated online, while the array is used for the analysis. The mask may be either generated offline or online.

The term "offline" as used herein is a broad term and is to be given its ordinary and customary meaning to a person of ordinary skill in the art and is not to be limited to a special or customized meaning. The term specifically may refer, without limitation, to generating the mask at a time before the array is used for analysis, e.g. at time of production or more or less right after production of the array, but always before the analysis. The mask may be pre-determined by one or more of inspection of every array after production, inspection of samples of a production lot, inspection before the array is used for the analysis such as by visual inspection, conducting and/or observing of a quality control while using the array. For example, the mask may be generated upon inspection of every array after production. Defects, such as molding artefacts may be added to the mask. Optionally surrounding regions may be added to the mask to be excluded. In this example, a mask for every array may be generated. For example, the mask may be generated upon inspection samples of a production lot, where the sample represent the lot. In this example, one mask per lot may be generated. For example, the mask may be generated upon inspection right before the array is used for the analysis, e.g. by visual inspection.

The method may comprise excluding the microfluidic chip or at least one subset of a batch of microfluidic chips from usage if a fraction of valid partitions is lower than a pre-defined minimum fraction of valid partitions after pre-determining the mask. The minimum fraction of valid partitions may depend on the analytical performance required. Many applications may easily tolerate 5% invalid partitions, some even 10 or 15%. Some systems may even tolerate more than 50% invalid partitions. Additionally or alternatively to the fraction, an absolute minimal number of required valid partitions may be used. For example, if the masking process yields less than 2'000 valid partitions, then the array may be invalidated.

The term "online" as used herein is a broad term and is to be given its ordinary and customary meaning to a person of ordinary skill in the art and is not to be limited to a special or customized meaning. The term specifically may refer, without limitation, to generating the mask is generated at least partially during using of the array. For example, the mask may be generated by conducting and/or observing of a quality control while using the array, whereby the signal of the quality control is detectable separately, e.g. in a separate fluorescence channel, and where the outcome of the quality control is sufficiently known to validate the elements of the array. For example, the method comprises conducting a digital polymerase chain reaction assay. A first fluorescence channel may be used for analysis of the sample and a second fluorescence channel may be used for pre-determining or generating the mask.

The mask may be an algorithmically defined mask. In the following, two embodiments of algorithmically defined mask will be described. These embodiments allow providing an encoding scheme that offers high flexibility to mask smaller or larger areas affected by systematic injection molding defects, using a small set of parameters. The shape of defect areas can be characterized by simple geometrical figures, such as rectangles or triangles, or a combination thereof. The extent of the affected area may still vary among injection molding lots, but this can be compensated by algorithmic modification of a general mask, e.g. scaling by a factor. Also, application of different masks within an injection molding run, e.g. due to increased wear of the stamper and subsequent increase of defect areas at exposed may be possible by an algorithmic approach. For example, the areas affected by injection molding defects may be predominantly located at the edges and corners of the rectangular arrays. For example, depending on the circumstances, defects may affect all lanes equally, or all lanes independently.

For example, mask parameters and partition indices of the mask may be stored in a software of the data processing unit and at least one corresponding identifier may be stored on a barcode of the microfluidic chip. The term "identifier" as used herein is a broad term and is to be given its ordinary and customary meaning to a person of ordinary skill in the art and is not to be limited to a special or customized meaning. The term specifically may refer, without limitation, to an element or a combination of elements configured for storing one or more items of information identifying the mask such as in a readable fashion, specifically in a machine-readable fashion. The identifier may comprise at least one of an optical identifier, an electronic identifier, a magnetic identifier or a mechanical identifier. As an example, the identifier, specifically the optical identifier, may be or may comprise at least one of a one- or two-dimensional code and/or a readable information tag, such as one or more of a barcode, a QR code or another type of code directly or indirectly attached to microfluidic chip such as by being applied directly to microfluidic chip and/or by being attached to microfluidic chip via at least one label or tag. The information stored in the identifier may be read using an appropriate reading device. This example may allow for more complex mask shapes, but only for a set of pre-defined masks; assuming the software on a sample processing system cannot be updated at all times. For example, the mask configurations may be stored on the barcode, e.g. as a 3 digit number (000 - 999). Thereby allowing for 1000 different masks. Mask configurations may then be stored in the software of the instrument. For example, the encoding may be performed using characters instead of numbers. For example, masks may be stored as series of partition coordinates (or partition indices) per lane. Each partition in the mask is excluded from the analysis. This example may allow for highest flexibility but requires most storage space. For example, masks may be stored as series of simple geometrical figures, e.g. as sets of rectangles and triangles. A rectangle can e.g. be defined by two corners, top-left and bottom-right, requiring only two partition indices to span up a rectangle. Similarly, a triangle can be spun up by only three partition indices. All partitions within these geometrical shapes may be subsequently masked.

For example, mask parameters may be directly stored in a barcode of the microfluidic device and indices of the partitions of the mask are calculated by a software of the data processing unit based on a model and the parameters from the barcode. This example may allow for any kind of mask but only within the possibility within the algorithmic model defined by the parameters. This example of defining masks may offer highest flexibility if the principle nature of defects within the injection molding process is well known in terms of location and geometry, but can vary in extent. In any case, the extend of affected area may have to be assessed during production for each lot and the information for subsequent masking has to be encoded on each microfluidic chip, e.g. on its barcode. For example, masks may be parameterized and stored as set of parameter on the barcode. The parameter model may be stored on the software of the sample processing system and may use the parameters read from the device barcode to reconstruct the mask.

The mask may be provided as one or more of: data on a data carrier such as a harddisk, a server, a cloud, a USB-Disk, CD, barcode, 2D-Barcode, QR-code, RFID; a downloadable file provided by a producer or provider of the array; data provided on the array being machine or human readable e.g. as text, 1D or 2D barcode, RFID; as data provided on a part attached to the array such as on a carrier being an assembly with the array; data, which can be looked up, by means of a reference e.g. by providing a human or machine-readable tag applied to the array or attached to the array or delivered with the array or by providing a reference such as a unique ID or identification number and using this reference to retrieve the mask e.g. by looking up the mask data e.g. by using the production-lot number, to look-up the mask related to the corresponding production lot e.g. by retrieving the mask data from a remote data storage location such as a cloud or a server by means of the reference; by uploading raw-data to a remote storage, wherefrom the data can get retrieved by the data processing unit; by pushing, mask data from a primary storage place, to a secondary storage place; by hosting, mask data on the data processing unit.

The term "barcode" as used herein is a broad term and is to be given its ordinary and customary meaning to a person of ordinary skill in the art and is not to be limited to a special or customized meaning. The term specifically may refer, without limitation, to binary optical information, such as to a binary sequence of optical information, such as a sequence of parallel lines having different widths, the binary sequence encoding information such as a number and/or an array of numbers and/or letters. Thus, the barcode may be a sequence of single colored lines having a high contrast compared to a background. Specifically, the barcode may comprise black lines on a white background.

The term "QR code" as used herein is a broad term and is to be given its ordinary and customary meaning to a person of ordinary skill in the art and is not to be limited to a special or customized meaning. The term specifically may refer, without limitation, to a quadratic matrix of binary pixels, the matrix encoding information such as a number and/or an array of numbers and/or letters. The pixels of the quadratic matrix may have a high contrast compared to a background. Specifically, the pixels of the matrix may comprise black squares arranged on a white background. Further, the QR code may comprise an indication of orientation enabling the reading device of the QR code to align the matrix.

The term "RFID tag" as used herein is a broad term and is to be given its ordinary and customary meaning to a person of ordinary skill in the art and is not to be limited to a special or customized meaning. The term specifically may refer, without limitation, to a label configured for exchanging data information with a reading device by using radio-frequency electromagnetic radiation, such as by using the NFC standard. The RFID tag may further comprise an antenna configured to receive and to emit a radio-frequency signal and an electronic chip such as a microchip configured to store the data information. Specifically, the RFID tag may be a flexible substrate having an electronic conducting coil and optionally at least one microchip.

For example, the mask may be provided as data on a data carrier, such as Harddisk, Server, Cloud, USB-Disk, CD, Barcode, 2D-Barcode, QR-code, RFID.

For example, the mask may be provided as a downloadable file provided by the producer and/or provider of the array.

For example, the mask may be provided as data provided on the array, being machine or human readable, e.g. as text, 1D or 2D barcode, RFID.

For example, the mask may be provided as data provided on a part attached to array, such as on carrier being an assembly with array.

For example, the mask may be provided as data, which can be looked up, by means of a reference, e.g. providing a human or machine-readable tag applied to the array or attached to the array or delivered with the array, providing a reference (unique ID, or identification number). The term "identification number" as used herein is a broad term and is to be given its ordinary and customary meaning to a person of ordinary skill in the art and is not to be limited to a special or customized meaning. The term specifically may refer, without limitation, to one or more of an array or a sequence of numbers and/or letters encoding the identification information of a specific mask. The identification number may be unique for a specific mask. Thus, it may be possible to identify a specific mask according to the respective identification number. This reference may be used for retrieving the mask data e.g. by looking up the mask data e.g. by using the production-lot number, to look-up the mask related to the corresponding production lot, e.g. by retrieving the mask data from a remote data storage location (such as e.g. cloud or server), by means of the reference.

For example, the mask may be provided by uploading raw-data to a remote storage, wherefrom the data can be retrieved by data-processing units for masking and data processing.

For example, the mask may be provided by pushing, mask data from a primary storage place, to a secondary storage place, where the mask data is either being used and/or processed or applied to array data.

For example, the mask may be provided by hosting, mask data on a data-processing unit which applies the mask to the array-data, where the data processing unit is a server- or a cloud-based solution.

The term "applying the mask" as used herein is a broad term and is to be given its ordinary and customary meaning to a person of ordinary skill in the art and is not to be limited to a special or customized meaning. The term specifically may refer, without limitation, to include, exclude and/or weighting the (optimally preprocessed) image data thereby generating masked data. The applying of the mask comprises masking each partition of the array depending on the mask.

The method may comprise post-processing the masked data. In the post-processing process similar steps may be executed as mentioned in the section pre-processing steps (e.g. binning, stitching, fluorescence cross-talk compensation, etc.), if not allocated in the pre-processing step.

The method may comprise flagging an analysis if a fraction of valid partitions is lower than a pre-defined minimum fraction of valid partitions after applying the mask. Additionally or alternatively, the method may comprise flagging an analysis if a number of valid partitions is lower than a pre-defined minimum number of valid partitions after applying the mask. This minimum fraction of valid partition may depend to the use-case (type of analyte) and the quality requirements. Many applications can tolerate 5, 10 or even 15% invalid partitions. The method may comprise providing at least one information depending on the flag to a user via at least one user-interface. The term "user interface" as used herein is a broad term and is to be given its ordinary and customary meaning to a person of ordinary skill in the art and is not to be limited to a special or customized meaning. The term may refer, without limitation, to an element or unit which is configured for interacting with its environment, such as for the purpose of unidirectionally or bidirectionally exchanging information, such as for exchange of one or more of data or commands. For example, the user interface may be configured to share information with a user and to receive information by the user. The user interface may be a feature to interact visually with a user, such as a display, or a feature to interact acoustically with the user. The user interface, as an example, may comprise one or more of: a graphical user interface; a data interface, such as a wireless and/or a wire-bound data interface.

The generating of the analytical result for the sample using said masked data may comprise evaluating the masked data. The term "evaluating" as used herein is a broad term and is to be given its ordinary and customary meaning to a person of ordinary skill in the art and is not to be limited to a special or customized meaning. The term specifically may refer, without limitation, to an application of methods for processing the masked data, e.g. at least one image evaluation algorithm, and deriving at least one representative result therefrom, such as a quantitative and/or qualitative result. This analytical results may be one or more of, a result for concentration of a distinct target analyte, a result for the presence or absence of an analyte, a result for a distinct copy-number-variation, a result for a distinct genetic mutation, a result for gene-expression, a result related to gene editing, etc.

For example, the method may comprise the following steps:
- providing an array
- providing a sample
- optionally, providing at least one reagents to analyze said sample on/in said array
- optionally, providing an instrument to analyze said sample on/in said array
- generating data from the sample by means of the array
- optionally pre-processing the data received from the array
- providing, such as retrieving or generating a mask relevant for the array
- masking the (optionally preprocessed) data from the sample
- optionally post-processing the masked data.
- generating at least one analytical result for the sample from said masked data.

In a further aspect, a sample processing system configured for processing a sample on at least one microfluidic chip is disclosed. The sample processing system is configured for performing a method for compensating defective partitions of a microfluidic chip according to the present invention, such as according to one or more of the embodiments given above or given in further detail below. For details, options and definitions, reference may be made to the description of the method.

The term "system" as used herein is a broad term and is to be given its ordinary and customary meaning to a person of ordinary skill in the art and is not to be limited to a special or customized meaning. The term may refer, without limitation, to an arbitrary set of interacting or interdependent components parts forming a whole. Specifically, the components may interact with each other in order to fulfill at least one common function. The components of the system may be handled independently or may be coupled or connectable.

The microfluidic chip comprises at least one array of partitions. The sample processing system comprises at least one imaging device configured for generating image data of the microfluidic chip comprising a sample by imaging the microfluidic chip. The sample processing system comprises at least one data processing unit configured for providing at least one mask for the microfluidic chip. The data processing unit is configured for applying the mask to one or more of the image data, at least one feature extracted from the image data, or at least one value extracted from the image data, thereby generating masked data. The applying of the mask comprises masking each partition of the array depending on the mask. The sample processing system is configured for generating at least one analytical result for the sample using said masked data.

Further disclosed and proposed herein is a computer program including computer-executable instructions for performing the method according to the present invention in one or more of the embodiments enclosed herein when the instructions are executed on a computer or computer network. Specifically, the computer program may be stored on a computer-readable data carrier and/or on a computer-readable storage medium.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one, more than one or even all of method steps a) to d) as indicated above may be performed by using a computer or a computer network, preferably by using a computer program.

Further disclosed and proposed herein is a computer program product having program code means, in order to perform the method according to the present invention in one or more of the embodiments enclosed herein when the program is executed on a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute the method according to one or more of the embodiments disclosed herein.

Further disclosed and proposed herein is a non-transient computer-readable medium including instructions that, when executed by one or more processors, cause the one or more processors to perform the method according to the present invention.

Further disclosed and proposed herein is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Finally, disclosed and proposed herein is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, further disclosed herein are:
- a computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

The method and means, presented here, may be therefore able
- to save production costs, production effort, reduce the number of optimization loops, and eases the control over the production parameters.
- to ease lot release
- to prolong the use time of injection molding tools to produce such the microfluidic chips,
- to, subsequentially, reduce costs using these chips to generate analytical results, and
- to reduce the number of discarded microfluidic chips, which otherwise would have to be discarded due to poor quality.
All above, without significantly compromising the analytical results or compromising the quality of the analytical result (such as precision, accuracy) generated with such microfluidic chips, even though they might not be perfect and have a significant fraction of defective partitions.

Summarizing and without excluding further possible embodiments, the following embodiments may be envisaged:
Embodiment 1. A method for compensating defective partitions of a microfluidic chip, wherein the microfluidic chip comprises at least one array of partitions, wherein the method comprises the following steps:
   a) generating image data of the microfluidic chip comprising a sample by imaging the microfluidic chip by using at least one imaging device;
   b) providing at least one mask for at least one area of partitions to at least one data processing unit;
   c) applying the mask, by using the data processing unit, to one or more of the image data, at least one feature extracted from the image data, or at least one value extracted from the image data, thereby generating masked data, wherein the applying of the mask comprises masking each partition of the array depending on the mask;
   d) generating at least one analytical result for the sample using said masked data.
Embodiment 2. The method according to the preceding embodiment, wherein the mask is a data set for inclusion, exclusion and/or weighting partitions of the array.
Embodiment 3. The method according to the preceding embodiment, wherein the weighting comprises applying weighting factors, wherein the weighting factor is a value between 0 and 1.
Embodiment 4. The method according to any one of the preceding embodiments, wherein the mask comprises a list of partitions and/or a range of partitions.
Embodiment 5. The method according to the preceding embodiment, wherein the range of partitions comprises one or more of a list of rows of the array, a list of columns of the array, a rim-size, a list of array areas, a list of predefined geometrical figures, such as rectangles or triangles, or a combination thereof.
Embodiment 6. The method according to any one of the preceding embodiments, wherein the mask is an array specific mask or a mask per lot.
Embodiment 7. The method according to any one of the preceding embodiments, wherein the providing of the mask comprises retrieving and/or generating the mask.
Embodiment 8. The method according to any one of the preceding embodiments, wherein the mask is pre-determined or is generated online while the array is used for the analysis.
Embodiment 9. The method according to the preceding embodiment, wherein the mask is pre-determined by one or more of inspection of every array after production, inspection of samples of a production lot, inspection before the array is used for the analysis such as by visual inspection, conducting and/or observing of a quality control while using the array.
Embodiment 10. The method according to the preceding embodiment, wherein the method comprises excluding the microfluidic chip or at least one subset of a batch of microfluidic chips from usage if a fraction of valid partitions is lower than a pre-defined minimum fraction of valid partitions after pre-determining the mask.
Embodiment 11. The method according to any one of the three preceding embodiments, wherein the method comprises conducting a digital polymerase chain reaction assay, wherein a first fluorescence channel is used for analysis of the sample and a second fluorescence channel is used for pre-determining or generating the mask.
Embodiment 12. The method according to any one of the preceding embodiments, wherein the mask is provided as one or more of: data on a data carrier such as a harddisk, a server, a cloud, a USB-Disk, CD, barcode, 2D-Barcode, QR-code, RFID; a downloadable file provided by a producer or provider of the array; data provided on the array being machine or human readable e.g. as text, 1D or 2D barcode, RFID; as data provided on a part attached to the array such as on a carrier being an assembly with the array; data, which can be looked up, by means of a reference e.g. by providing a human or machine-readable tag applied to the array or attached to the array or delivered with the array or by providing a reference such as a unique ID or identification number and using this reference to retrieve the mask e.g. by looking up the mask data e.g. by using the production-lot number, to look-up the mask related to the corresponding production lot e.g. by retrieving the mask data from a remote data storage location such as a cloud or a server by means of the reference; by uploading raw-data to a remote storage, wherefrom the data can get retrieved by the data processing unit; by pushing, mask data from a primary storage place, to a secondary storage place; by hosting, mask data on the data processing unit.
Embodiment 13. The method according to any one of the preceding embodiments, wherein the mask is an algorithmically defined mask, wherein mask parameters and partition indices of the mask are stored in a software of the data processing unit and at least one corresponding identifier is stored on a barcode of the microfluidic chip, or wherein mask parameters are directly stored in a barcode of the microfluidic chip and indices of the partitions of the mask are calculated by a software of the data processing unit based on a model and the parameters from the barcode.
Embodiment 14. The method according to any one of the preceding embodiments, wherein the method comprises flagging an analysis if a fraction and/or number of valid partitions is lower than a pre-defined minimum fraction and/or minimum number of valid partitions after applying the mask.
Embodiment 15. The method according to any one of the preceding embodiments, wherein the method comprises providing of the microfluidic chip, wherein the method comprises at least one manufacturing process, wherein the manufacturing process comprises injection molding.
Embodiment 16. The method according to any one of the preceding embodiments, wherein the method comprises at least one partitioning step in which a sample is filled into the microfluidic chip, wherein the method comprises providing at least one reagent to analyze said sample on said microfluidic chip.
Embodiment 17. The method according to any one of the preceding embodiments, wherein the method comprises providing an instrument to analyze said sample on said microfluidic chip.
Embodiment 18. The method according to any one of the preceding embodiments, wherein the method comprises preprocessing the image data.
Embodiment 19. The method according to any one of the preceding embodiments, wherein the method comprises post-processing the masked data.
Embodiment 20. The method according to any one of the preceding embodiments, wherein the data processing unit is a server- or a cloud-based solution.
Embodiment 21. The method according to any one of the preceding embodiments, wherein the method is computer-implemented.
Embodiment 22. A sample processing system configured for processing a sample on at least one microfluidic chip, wherein the sample processing system is configured for performing a method for compensating defective partitions of a microfluidic chip according to any one of the preceding embodiments, wherein the microfluidic chip comprises at least one array of partitions, wherein the sample processing system comprises at least one imaging device configured for generating image data of the microfluidic chip comprising a sample by imaging the microfluidic chip, wherein the sample processing system comprises at least one data processing unit configured for providing at least one mask for the microfluidic chip, wherein the data processing unit is configured for applying the mask to one or more of the image data, at least one feature extracted from the image data, or at least one value extracted from the image data, thereby generating masked data, wherein the applying of the mask comprises masking each partition of the array depending on the mask, wherein the sample processing system is configured for generating at least one analytical result for the sample using said masked data.
Embodiment 23. A computer program comprising instructions which, when the program is executed by the sample processing system according to the preceding embodiment, cause the sample processing system to perform the method according to any one of the preceding embodiments referring to a method.
Embodiment 24. A computer-readable storage medium comprising instructions which, when the instructions are executed by the sample processing system according to embodiment 23, cause the sample processing system to perform the method according to any one of the preceding embodiments referring to a method.
Embodiment 25. A non-transient computer-readable medium including instructions that, when executed by one or more processors, cause the one or more processors to perform the method according to any one of the preceding embodiments referring to a method.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows an embodiment of a sample processing system in a schematic view. The microfluidic chip comprises 4 arrays of partitions;
- Figure 2: shows an embodiment of a method for compensating defective partitions of a microfluidic chip;
- Figures 3A and 3B: show embodiments of a microfluidic chip;
- Figure 4: shows embodiments of predefined masks; and
- Figures 5A to 5C: show embodiments of mask parameters and partition indices on a microfluidic chip.

### Detailed description of the embodiments

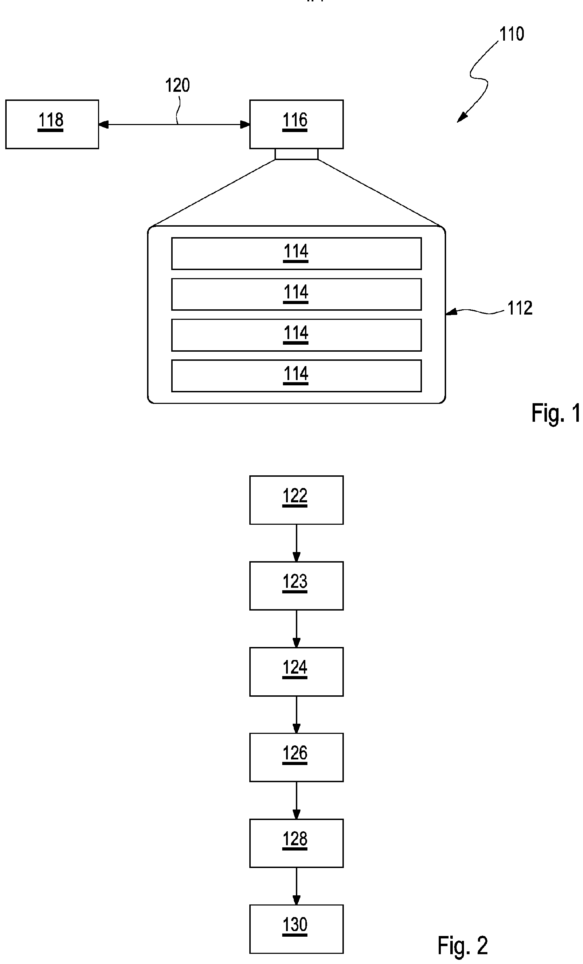

Figure 1 shows an exemplary embodiment of a sample processing system 110 in a schematic view. The sample processing system is configured for processing a sample (not shown in the Figures) on at least one microfluidic chip 112. The microfluidic chip 112 comprises at least one array of partitions 114. In this example, the microfluidic chip 112 comprises four rectangular arrays of partitions 114, also denoted as lanes of array of partitions 114. However, other geometrical forms of the arrays of partitions 114 and/or another number of arrays of partitions comprised by the microfluidic chip are also feasible.

The sample processing system comprises at least one imaging device 116 configured for generating image data of the microfluidic chip 112 comprising a sample by imaging the microfluidic chip 112. The sample processing system further comprises at least one data processing unit 118 configured for providing at least one mask for the microfluidic chip 116. As indicated by arrow 120 in Figure 1, the imaging device 116 and the data processing unit 118 may be configured for unidirectionally or bidirectionally exchanging data, specifically image data, for example via a data exchange interface. The data processing unit may, as an example, be a server- or a cloud-based solution.

The data processing unit 118 is configured for applying the mask to one or more of the image data, at least one feature extracted from the image data, or at least one value extracted from the image data, thereby generating masked data. The applying of the mask comprises masking each partition of the array 114 depending on the mask. The sample processing system 110 is configured for generating at least one analytical result for the sample using said masked data.

The sample processing system 110 is further configured for performing a method for compensating defective partitions of a microfluidic chip 112 according to the present invention, such as according to the embodiment of the method shown in Figure 2. Thus, for a description of the method, reference is made to the description of Figure 2.

Figure 2 shows an exemplary embodiment of a method for compensating defective partitions of a microfluidic chip 112. As shown in Figure 1, the microfluidic chip 112 comprises at least one array of partitions, e.g. a plurality of arrays of partitions 114. The method may specifically be performed by the sample processing system 110 shown in Figure 1.

The method may comprise at least one partitioning step (denoted by reference number 122) in which a sample is filled into the microfluidic chip 112. For example, a solution comprising analytes of interest may be physically distributed into the partitions. The method further may comprise providing at least one reagent to analyze said sample on said microfluidic chip 112. For example, the microfluidic chip 112 is configured for digital PCR or biochemical assaying of a sample provided, e.g. in the form of a reaction mixture, to each of the partitions by means of the microfluidic devices.

The method may comprise at least one reaction step (denoted by reference number 123) in which a chemical, biochemical, or biological or physical reaction takes place, especially if an analyte is present or not present in any of partitions of the array. E.g. conducting d-PCR or a d-isothermal nucleic acid amplification reaction or a d-enzymatic reaction.

Further, the method comprises the following steps:
a) (denoted by reference number 124) generating image data of the microfluidic chip 112 comprising the sample by imaging the microfluidic chip 112 by using the at least one imaging device 116;
b) (denoted by reference number 126) providing at least one mask for at least one area of partitions to the at least one data processing unit 118;
c) (denoted by reference number 128) applying the mask, by using the data processing unit 118, to one or more of the image data, at least one feature extracted from the image data, or at least one value extracted from the image data, thereby generating masked data, wherein the applying of the mask comprises masking each partition of the array 114 depending on the mask;
d) (denoted by reference number 130) generating at least one analytical result for the sample using said masked data.

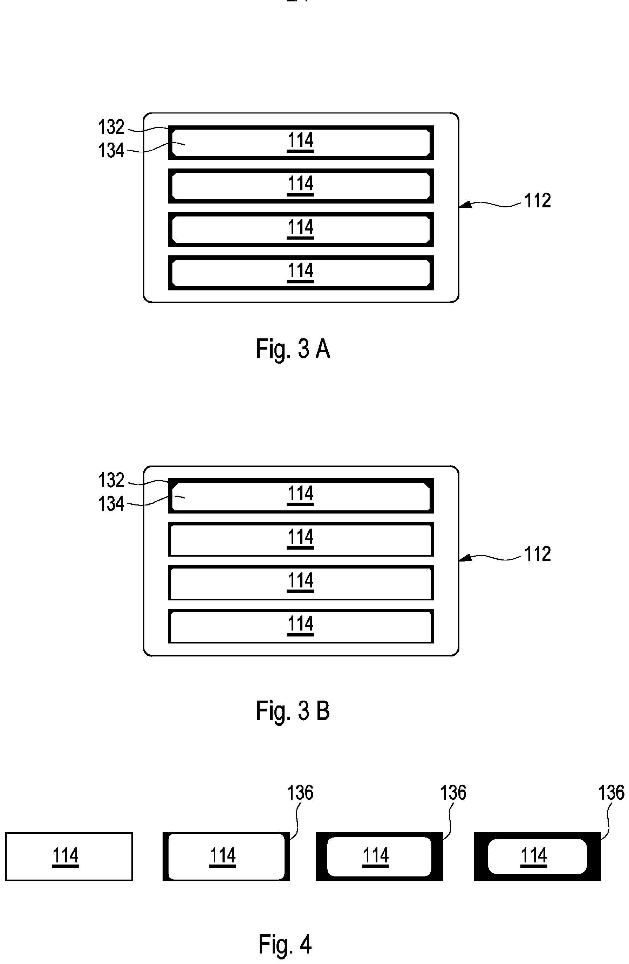

It is generally known that microfluidic chips 112 may suffer from quality issues in the manufacturing process at specific spatial locations due to the nature of the manufacturing process. The manufacturing process may result in defect and/or non-valid partitions of the microfluidic chip 112. These defect and/or non-valid partitions would negatively affect the analytical results if the analysis is performed on those defect partitions. Exemplary embodiment of the microfluidic chip 112 with defects and/or non-valid partitions is shown in Figures 3A and 3B. In Figures 3A and 3B, defect and/or non-valid partitions of the microfluidic chip 112 are indicated by reference number 132, whereas areas without defects are indicated by reference number 134. The areas 132, 134 affected by injection molding defects may be predominantly located at the edges and corners of the rectangular arrays 114. As shown in Figure 3A, defects may affect all lanes equally or, as shown in Figure 3B, all lanes independently. The method according to the present invention, for example according to the embodiment shown in Figure 2, may allow for reliably excluding areas with defects 132 from the analysis. The method according to the present invention, for example according to the embodiment shown in Figure 2, may further allows for not excluding large areas with valid partitions 134 (partitions with no defects).

Turning back to Figure 2, the providing of the mask in step b) (denoted by reference number 126) may comprise retrieving and/or generating the mask. The mask may be pre-determined or may be generated online while the array is used for the analysis. The mask may be either generated offline or online.

As an example, Figure 4 shows exemplary embodiments of pre-determined masks 136. The mask may be pre-determined by one or more of inspection of every array 114 after production, inspection of samples of a production lot, inspection before the array 114 is used for the analysis such as by visual inspection, conducting and/or observing of a quality control while using the array 113. For example, the mask may be generated upon inspection of every array 114 after production. Defects, such as molding artefacts may be added to the mask. Optionally surrounding regions may be added to the mask to be excluded. In this example, a mask for every array 114 may be generated. For example, the mask may be generated upon inspection samples of a production lot, where the sample represent the lot. In this example, one mask per lot may be generated. For example, the mask may be generated upon inspection right before the array is used for the analysis, e.g. by visual inspection.

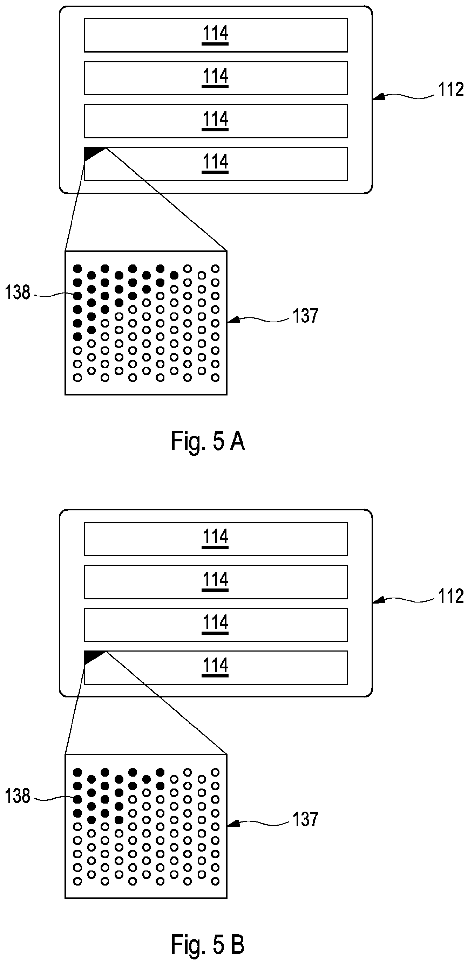

Alternatively or additionally to pre-determined masks, the mask may be an algorithmically defined mask. Exemplary embodiments of algorithmically defined masks are shown in Figures 5A to 5C. Specifically, Figures 5A to 5C show different exemplary embodiments of mask parameters and partition indices on the microfluidic chip 112. These embodiments allow providing an encoding scheme that offers high flexibility to mask smaller or larger areas affected by systematic injection molding defects, using a small set of parameters. The shape of defect areas can be characterized by simple geometrical figures, such as rectangles or triangles, or a combination thereof. The extent of the affected area may still vary among injection molding lots, but this can be compensated by algorithmic modification of a general mask, e.g. scaling by a factor. Also, application of different masks within an injection molding run, e.g. due to increased wear of the stamper and subsequent increase of defect areas at exposed may be possible by an algorithmic approach.

Mask parameters and partition indices of the mask 137 may be stored in a software of the data processing unit 118 and at least one corresponding identifier may be stored on a barcode (not shown in the Figures) of the microfluidic chip 112. The information stored in the identifier may be read using an appropriate reading device. This example may allow for more complex mask shapes, but only for a set of pre-defined masks; assuming the software on a sample processing system 110 cannot be updated at all times. For example, the mask configurations may be stored on the barcode, e.g. as a 3 digit number (000 - 999). Thereby allowing for 1000 different masks. Mask configurations may then be stored in the software of the instrument. For example, the encoding may be performed using characters instead of numbers.

As shown in Figure 5A, masks may be stored as series of partition coordinates (or partition indices) per lane. Each partition in the mask is excluded from the analysis. A part of an exemplary mask 137 for lane 4 of the microfluidic chip 112 is shown in the enlarged view of Figure 5A. Therein, dark points indicate partition coordinates which are excluded from the analysis (denoted by reference number 138), wherein other, non-marked partition coordinate are used for the analysis. In this example, the mask 137 may comprise for lane 4 the following list of partitions of indices: 0, 1, 2, 3, 4, 5, 50, 51, 52, 53, 54, 100, 101, 102, 103, 150, 151, 152, 201, 202, 203, 250, 251, 300, 301, 400. This example may allow for highest flexibility but requires most storage space.

Alternatively or additionally, as shown in Figure 5B, masks may be stored as series of simple geometrical figures, e.g. as sets of rectangles and triangles. A rectangle can e.g. be defined by two corners, top-left and bottom-right, requiring only two partition indices to span up a rectangle. Similarly, a triangle can be spun up by only three partition indices. All partitions within these geometrical shapes may be subsequently masked. The enlarged view of Figure 5B shows a part of another exemplary mask 137 for lane 4 of the microfluidic chip 112. In this example, the mask 137 comprises at the top left masked area two rectangles being defined by corners top-left and bottom-right at partition indices [0, 153] and [200, 301].

As another example, mask parameters may be directly stored in a barcode of the microfluidic device and indices of the partitions of the mask 137 are calculated by a software of the data processing unit 118 based on a model and the parameters from the barcode. This example may allow for any kind of mask 137 but only within the possibility within the algorithmic model defined by the parameters. This example of defining masks 137 may offer highest flexibility if the principle nature of defects within the injection molding process is well known in terms of location and geometry, but can vary in extent. In any case, the extend of affected area may have to be assessed during production for each lot and the information for subsequent masking has to be encoded on each microfluidic chip 112, e.g. on its barcode. For example, masks 137 may be parameterized and stored as set of parameter on the barcode.

The parameter model may be stored on the software of the sample processing system and may use the parameters read from the device barcode to reconstruct the mask.

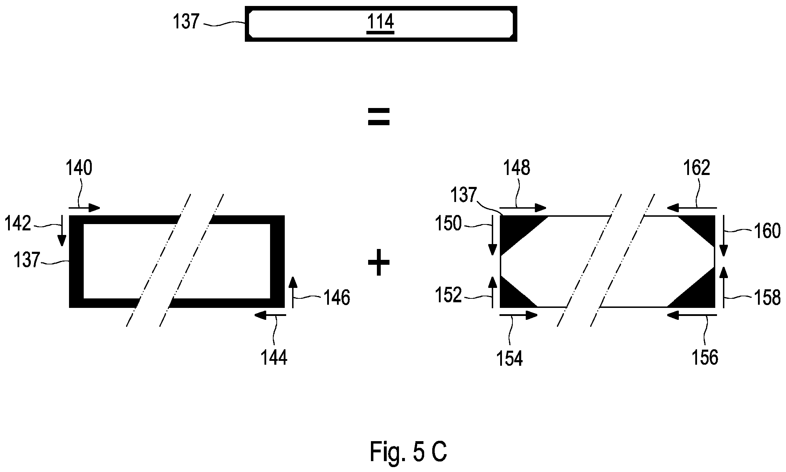

As shown in Figure 5C, a mask 137 covering typical locations on the rectangular array of partitions 114 shall be algorithmically defined. The mask 137 shall cover the left, right, top and bottom border areas, defined as rectangles, and triangles in each corner of the rectangular arrays of partitions 114. In this example, a number of twelve parameters is to be stored per lane. As shown Figure 5C, the parameter comprise four parameters to define the four rectangles, i.e. a number of columns from the left (denoted by reference number 140), a number of rows from the top (denoted by reference number 142), a number of columns from the right (denoted by reference number 144) and a number of rows from the bottom (denoted by reference number 146), and eight parameters to define the four triangles, i.e. a number of columns from the left (denoted by reference number 148), a number of rows from the top (denoted by reference number 150), a number of rows from the bottom (denoted by reference number 152), a number of columns from the left (denoted by reference number 154), a number of columns from the right (denoted by reference number 156), a number of rows from the bottom (denoted by reference number 158), a number of rows from the top (denoted by reference number 160) and a number of columns from the right (denoted by reference number 162). In case the triangles are isosceles triangles, the number of parameters may be reduced to eight values per lane. In case of even higher symmetry, the number of parameters may be further reduced to parameterize the masks.

Turning back to Figure 2, the method may comprise further processing steps with the masked data. For example, the method may comprise flagging an analysis if a number of valid partitions is lower than a pre-defined minimum fraction of valid partitions after applying the mask, e.g. if for example more than 15% of the partitions are lost due to masking. Alternatively or additionally, the method may comprise providing at least one information depending on the flag to a user via at least one user-interface. Alternatively or additionally, the generating of the analytical result for the sample using said masked data may comprise evaluating the masked data.

The following examples further illustrate the proposed method:

### Example 1: Providing and using batch-specific mask

### Step A: Produce a batch (lot) of microarrays

A batch of dPCR-consumables, Type "A", is produced. The dPCR consumable has the following aspects.

| Aspect | Type "A" |
|---|---|
| Area of cavities [mm2] | 6x85 |
| Number of cavities per device [1] | 20k |
| Volume per cavity, [nl] | 2.5 |
| Shape of cavity, arrangement | Hexagonal, honeycomb |
| Length of cavity , [mm] | 0.2 |
| Width of cavity [mm] | 0.1 |
| Depth of cavity, [mm] | 0.16 |
| Open depth of void, [mm] | 0.02 to 0.25 |
| Type of Coating | Silica |
| Method of production | Injection molding of body Punched cover foil Thermally sealed |
| Material | Cyclic Olefin-Polymer |

The batch had a size of 2012 consumables. All consumables were produced form 1 tool (mold) and 1 assembly line. The consumables of the batch had a batch-ID and a run-number from "0001" to "2012".

### Step B: Take samples from batch

Form the production, 12 samples got analyzed for batch release: The following samples were analyzed: 4 samples: from the beginning of the production batch, 4 samples: from the middle of the production batch, 4 samples: from the end of the production batch.

### Step C: Analyze samples from batch

The samples from Example "1" have been analyzed by any of the following methods:

| **Example** | **Preprocess** | **Assembled** | **Analysis-Process** | **Integrity check** |
|---|---|---|---|---|
| 1A | None | Optional | Visual inspection, with a scanning microscope with | Pattern recognition of regular/non regular sections. |
| | | | A magnification in a range 1 to 20X | Non-regular being a results of any of: |
| | | | Scanning in X and/or Y-direction. | Dust, dirt, defect of tool, material accumulation from using the tool, wear out of tool, tool damages, and artefacts from tool manufacturing. |
| | | | Optionally being a line camera scanning only in on direction. | |
| | | | | Mark irregular partitions. |
| 1B | None | Optional | 1A using on top polarization filters | As 1A |
| 1C | None | Optional | 1A | As 1A |
| | | | A series of z-Positions are scanned to get better understanding on the 3D-structure | |
| 2A | Filled with a fluorescent sample such as 20nM Fluorescein, partitioned | Yes | Fluorescence imaging | Homogeneity of filled partitions. |
| | | | | Mark dim or bright wells. |
| | | | | Mark partitions, which are not fully separated by each other. |
| 2B | Filled with an aqueous buffer, partitioned | Yes | Imaged in the NIR, e.g. a a wavelength where water shows a strong absorption, e.g. at a wavelength of 1550nm using a IR-sensitive camera | Homogeneity of filling of the partitions. |
| | | | | Mark partitions, which are not fully separated by each other. |
| 2C | Filled with a dye solution such as 1g/l Methylene blue in H2O, partitioned | Yes | Inspect partitions in a transmission mode setting | Check for full separation of partitions. |
| | | | | Mark partitions, which are not fully separated by each other. |

| Example | Preprocess | Assem- | Analysis-Process | Integrity check |
|---|---|---|---|---|
| 3A | Filled with a positive dPCR master mix, preferably creating a defined positivity rate in a range of 0.05 to 0.7, preferably in a range of 0.1 to 0.5. | Yes | Conduct a dPCR. After PCR acquire fluorescence image in the relevant fluorescence channel, were positive reactions create a fluorescence. | Analyzes positivity-rate over the whole consumable, mark areas with elevated or reduced positivity rate. |
| | | | | Analyze brightness distribution of positive/ negative partitions over the whole consumable, and mark areas, which are not typical. |
| | Using a fluorescent probe such as as Taqman probe or an interchelatordye. | | | |
| 3B | Filled with a isothermal mastermix, such as for Rolling Circle Amplification (RCA), and a target producing the positivity rate fo 0.05 to 0.7, preferably 0.1 to 0.5. | | Conduct a isothermal reaction. Afterwards acquire a fluorescence images in the relevant channel | As 3A |
| | Using a fluorescent probe, such as a molecular beacon. | | | |

### Step D: Create the "mask", a list of all valid/non-valid partitions

Create a list of all non-valid partitions or areas on the consumable batch, using the results from the samples from Step B to Step C. Preferably an OR operation is used between the 12 samples (any non-valid partition in any of the samples will show up in the non-valid partitions for the batch).

### Step E: Store mask

Store the list of non-valid partitions ("Mask") in a way it is clear this mask relates to the corresponding production batch.

### Step F: Use consumable form the batch

Use a consumable from the production batch form Step A in an assay. Generate raw data for all partitions.

### Step G: Apply mask

Remove non-valid portions form the raw-data using the mask from Step E.

### Step H: Used masked data

Calculate a result based on the masked data from Step G.

The following steps may be further used optionally in this example:
- The consumable of Step A may be marked with a batch specific identifier (BID). The BID may be directly applied to the consumable, e.g. laser marked, or a label may carry the BID, being attached to the consumable, the label with the BID may be applied to a holder of the consumable. The BID may also only be applied to the package or bag of the consumable.
- The BID is later used to retrieve the mask related to the corresponding production batch.
- The mask form Step E may be stored in a way that it can be retrieved later using BID.
- The mask may be generated as part of a production QC and be generated by the producer of the consumable
- The mask may be stored locally or on a remote storage-device (e.g. cloud drive).
- The remote storage device may be set-up in a way that the mask-data is (only) accessible by the later user/consumer of the consumable.
- The mask may also be provided on paper, e.g. as 1D or 2D barcode, QR-code, which needs to be scanned before usage
- The mask can also be provided on a mobile storage device such as a CD or an USB-stick, provided with shipments of the consumable.
- The mask data may also be compressed. E.g listing complete areas, which are non-valid instead of a list of single non-valid partitions.
- Exclude batches or subset of consumables form usage if not a minimum fraction of valid partitions is present after masking.
- Flag an analysis if a minimum fraction of valid partitions is not present after masking.

### Example 2: Providing and using Mask form a Control-Run

### Step A: Defining a Subset of consumables from a production lot

Define 54 samples from a production lot, as a subset, being used in a study.

### Step B : Define samples

Define 4 consumables (of the 54 from above) as samples.

### Step C: Analyzes Samples

Analyze the 4 sample-consumables from Step B by any method as above, preferably by means of a QC-kit, by method 3A, producing a dPCR data with predefined positivity rate in range of 0.1 to 0.5.

### Step D: Create list of all valid/non-valid partitions (a "mask").

Create a list of all non-valid partitions or areas of the consumables subset from Step A, from the 4 samples, analyzed in C, using the results from the 4 samples from Step C. -Preferably an OR operation is used between the 4 samples. Any non-valid partition in any of the 4 samples will show up in the non-valid partitions mask, for the subset of consumables from Step A.

### Step E: Store mask

Store the list of non-valid partitions ("Mask") in a way it is clear this mask relates to the corresponding subset of consumables.

The mask data may be stored locally on local storage device, a local network-drive or non-local network-drive (such as a cloud storage). The data is stored in a way that the data can be easily found, accessed, retrieved, and applied if required.

### Step F:

Use a consumables from the Subset form Step A in an assay (Set of assays) Generate raw data for all partitions.

### Step G:

Remove non-valid portions form the raw-data using the mask from Step E. (Step F and G can also be combined in one step).

### Step H:

Calculate a result based on the masked data from Step G.

### Example 3 : Providing and using Mask for a System

### Step A: Developing a system having at least one consumable

- Developing a system having at least one consumable, used for partitioned analysis (e.g. used for dPCR).
- Produce at least one batch of the consumables, preferably produce multiple batches of the consumable.

### Step B : Take samples from produced consumables

Take a number of samples from the produced batches of consumable.

### Step C: Analyzes Samples

Analyze the sample-consumables from Step B by any method as above methods 1A to 3B.

### Step D: Create list of all valid/non-valid partitions (a "mask").

Create a list of all non-valid partitions or areas of the consumable. Derivate a mask covering all non-valid partitions of the consumable. Preferably an OR operation can used between all samples. Any non-valid partition in any sample will show up in the non-valid partitions mask, for the system according to Step A. Or generate mask, where only frequent errors are masked.

### Step E: Store mask

Store the list of non-valid partitions ("Mask") in a way it can get applied in the future by the system. Store the mask locally to the system conducting the analysis.

### Step F:

Use a consumable of the system. Generate raw data for all partitions.

### Step G:

Remove non-valid portions form the raw-data using the mask from Step D and E. (Step F and G can also be combined in one step).

### Step H:

Calculate a result based on the masked data from Step G.

### Example 4 : "Online" Generation of a Mask

### Step A: Taking an arrayed dPCR consumable

### Step B:

Conducting a dPCR analysis generating fluorescence in 2 independent fluorescent channels.
1. Where the FAM-Channel is used to analysis an unknown sample
2. Where the HEX-Channel is used to analysis the quality to the array, to determine thereafter the mask

In the HEX channel a target is analyzed of known composition and concentration, giving an expected result (e.g. generating a pattern, where in an average 50% of the elements have positive signal, and where in an average 50% of the elements generate a negative signal.

### Step C: Create list of all valid/non-valid partitions (a "mask").

After the dPCR reaction has been conducted, by locally analyzing the positive rate in the HEX channel, determine if the locally analyzed area is regular or not.

Add elements to the mask
- When all elements within the observed area are positive
- When all elements within the observe area are negative
- When the ratio of positive/negative elements significantly deviates from what is expected
- When the brightness of the positive or negative signals are not within expected ranges. The locally analyzed area can e.g. a patch of 5 x 5 element, or 10 x 10 elements or 3 x 8 elements or Ix 10 elements.

### Step E: Store mask

Store the list of non-valid partitions ("Mask") in a way it can get applied in the future by the system. E.g. store the mask locally to the system conducting the analysis.

### Step F:

Remove non-valid partitions form the raw-data in the FAM-Channel using the mask from C

### Step H:

Calculate a result based on the masked data from Step F

### List of reference numbers

- 110: sample processing system
- 112: microfluidic chip
- 114: array of partitions
- 116: imaging device
- 118: data processing unit
- 120: arrow
- 122: partitioning step
- 123: reaction step
- 124: generating image data
- 126: providing at least one mask
- 128: applying the mask
- 130: generating at least one analytical result
- 132: areas with defects and/or non-valid partitions
- 134: areas without defects
- 136: pre-defined mask
- 137: mask
- 138: partition coordinates which are excluded from the analysis
- 140: columns from the left
- 142: rows from the top
- 144: columns from the right
- 146: rows from the bottom
- 148: columns from the left
- 150: rows from the top
- 152: rows from the bottom
- 154: columns from the left
- 156: columns from the right
- 158: rows from the bottom
- 160: rows from the top
- 162: columns from the right

## Claims

1. A method for compensating defective partitions of a microfluidic chip (112), wherein the microfluidic chip (112) comprises at least one array of partitions (114), wherein the method comprises the following steps:
a) generating image data of the microfluidic chip (112) comprising a sample by imaging the microfluidic chip (112) by using at least one imaging device (116);
b) providing at least one mask (136, 137) for at least one area of partitions to at least one data processing unit (118);
c) applying the mask (136, 137), by using the data processing unit (118), to one or more of the image data, at least one feature extracted from the image data, or at least one value extracted from the image data, thereby generating masked data, wherein the applying of the mask comprises masking each partition of the array (114) depending on the mask;
d) generating at least one analytical result for the sample using said masked data.

2. The method according to the preceding claim, wherein the mask (136, 137) is a data set for inclusion, exclusion and/or weighting partitions of the array.

3. The method according to any one of the preceding claims, wherein the mask (136, 137) comprises a list of partitions and/or a range of partitions.

4. The method according to any one of the preceding claims, wherein the mask (136, 137) is an array specific mask or a mask per lot.

5. The method according to any one of the preceding claims, wherein the providing of the mask (136, 137) comprises retrieving and/or generating the mask (136, 137).

6. The method according to any one of the preceding claims, wherein the mask (136, 137) is pre-determined or is generated online while the array is used for the analysis.

7. The method according to the preceding claim, wherein the method comprises conducting a digital polymerase chain reaction assay, wherein a first fluorescence channel is used for analysis of the sample and a second fluorescence channel is used for pre-determining or generating the mask (136, 137).

8. The method according to any one of the preceding claims, wherein the mask (136, 137) is provided as one or more of: data on a data carrier such as a harddisk, a server, a cloud, a USB-Disk, CD, barcode, 2D-Barcode, QR-code, RFID; a downloadable file provided by a producer or provider of the array (114); data provided on the array (114) being machine or human readable e.g. as text, 1D or 2D barcode, RFID; as data provided on a part attached to the array (114) such as on a carrier being an assembly with the array (114); data, which can be looked up, by means of a reference e.g. by providing a human or machine-readable tag applied to the array (114) or attached to the array (114) or delivered with the array (114) or by providing a reference such as a unique ID or identification number and using this reference to retrieve the mask (136, 137) e.g. by looking up the mask data e.g. by using the production-lot number, to look-up the mask (136, 137) related to the corresponding production lot e.g. by retrieving the mask data from a remote data storage location such as a cloud or a server by means of the reference; by uploading raw-data to a remote storage, wherefrom the data can get retrieved by the data processing unit; by pushing, mask data from a primary storage place, to a secondary storage place; by hosting, mask data on the data processing unit.

9. The method according to any one of the preceding claims, wherein the mask (136, 137) is an algorithmically defined mask, wherein mask parameters and partition indices of the mask (136, 137) are stored in a software of the data processing unit (118) and at least one corresponding identifier is stored on a barcode of the microfluidic chip, or wherein mask parameters are directly stored in a barcode of the microfluidic chip and indices of the partitions of the mask are calculated by a software of the data processing unit based on a model and the parameters from the barcode.

10. The method according to any one of the preceding claims, wherein the method comprises flagging an analysis if a fraction and/or number of valid partitions is lower than a pre-defined minimum fraction and/or minimum number of valid partitions after applying the mask (136, 137).

11. The method according to any one of the preceding claims, wherein the method is computer-implemented.

12. A sample processing system (110) configured for processing a sample on at least one microfluidic chip (112), wherein the sample processing system (110) is configured for performing a method for compensating defective partitions of a microfluidic chip (112) according to any one of the preceding claims, wherein the microfluidic chip (112) comprises at least one array of partitions (114), wherein the sample processing system (110) comprises at least one imaging device (116) configured for generating image data of the microfluidic chip (112) comprising a sample by imaging the microfluidic chip (112), wherein the sample processing system (110) comprises at least one data processing unit (118) configured for providing at least one mask (136, 137) for the microfluidic chip (112), wherein the data processing unit (118) is configured for applying the mask (136, 137) to one or more of the image data, at least one feature extracted from the image data, or at least one value extracted from the image data, thereby generating masked data, wherein the applying of the mask (136, 137) comprises masking each partition of the array (114) depending on the mask (136, 137), wherein the sample processing system (110) is configured for generating at least one analytical result for the sample using said masked data.

13. A computer program comprising instructions which, when the program is executed by the sample processing system (110) according to the preceding claim, cause the sample processing system (110) to perform the method according to any one of the preceding claims referring to a method.

14. A computer-readable storage medium comprising instructions which, when the instructions are executed by the sample processing system (110) according to claim 12, cause the sample processing system (110) to perform the method according to any one of the preceding claims referring to a method.

15. A non-transient computer-readable medium including instructions that, when executed by one or more processors, cause the one or more processors to perform the method according to any one of the preceding claims referring to a method.
